# EUROPÄISCHE PATENTANMELDUNG

(11) **EP 4 215 177 A1**
(43) Veröffentlichungstag der Anmeldung: **26.07.2023**
(21) Anmeldenummer: 22197476.9
(22) Anmeldetag: 23.09.2022
(51) Int. Cl.: A61K 8/49, A61K 8/92, A61Q 5/00, A61Q 5/02, A61Q 5/12

(54) **HAARPFLEGEPRODUKTE OHNE SILIKONÖL UND MIT NATÜRLICHEN ÖLEN UND WACHSEN**

(30) Priorität: 30.11.2021 DE 102021131466
(71) Anmelder: Henkel AG & Co. KGaA, 40589 Düsseldorf (DE)
(72) Erfinder: von Aspern, Edith, 21271 Hanstedt (DE); Kerl, Sylvia, 25474 Bönningstedt (DE); Glasing, Joe, 22763 Hamburg (DE)

(57) **Zusammenfassung**

Die vorliegende Anmeldung betrifft eine silikonfreie kosmetische Zusammensetzung zur Pflege keratinischen Materials, insbesondere zur Pflege menschlicher Haare, die eine hohe Menge eines oder mehrerer natürlicher Wache und eine hohe Menge einer Ölkomponente umfasst.

## Beschreibung

Die vorliegende Anmeldung betrifft eine silikonfreie kosmetische Zusammensetzung zur Pflege keratinischen Materials, insbesondere zur Pflege menschlicher Haare, die eine hohe Menge eines oder mehrerer natürlicher Wache und eine hohe Menge einer Ölkomponente umfasst.

Haarspülungen und Shampoos mit pflegender Wirkung enthalten häufig Silikonöle, Fettsäuren und Polymere. Herkömmliche Produkte im Bereich der Haarkosmetik betreffen Haaröle oder Conditioner, bei denen die Pflegekomponente hauptsächlich aus Silikonölen besteht.

Im Rahmen der Nachhaltigkeit wird derzeit verstärkt Wert auf Rohstoffe pflanzlichen Ursprungs oder mit guter biologischer Abbaubarkeit gelegt. In diesem Zusammenhang sind Silikonöle in der öffentlichen Kritik. Ebenso verhält es sich bei polymeren Bestandteilen. Hier wird Kritik am Eintrag von Mikroplastik in die Umwelt geübt. Bei der Suche nach Alternativen soll das gute Pflegeniveau jedoch nicht beeinträchtigt werden. Eine große Schwierigkeit beim Austausch von Silikonölen in Haarkosmetika besteht darin, natürliche Ole und andere natürliche Rohstoffe zu finden, die das Haar nicht belasten aber trotzdem pflegen. Zudem besteht zunehmend Bedarf an kosmetischen Produkten, die nach Naturkosmetik-Richtlinie formuliert werden. Der Ruf nach Nachhaltigkeit auch durch Verwendung von nachhaltigen Rohstoffen wird kontinuierlich größer.

Der Bedarf an hervorragenden Pflegeprodukten ist oft einer gestiegenen Belastung der Haare geschuldet. Neben den natürlichen Umwelteinflüssen ist das menschliche Haar auch einer Reihe weiterer, insbesondere kosmetischer Belastungen ausgesetzt. Zu diesen Belastungen, die das Haar schädigen, gehören beispielsweise das Färben der Haare und die Haarformung, z.B. durch permanente Wellen. Kosmetische Haarpflegemittel werden eingesetzt, um die nachteiligen Auswirkungen der (Umgebungs-)Einflüsse, die die Haarstruktur beeinträchtigen, zu reduzieren, aber auch, um die natürliche Haarstruktur zu erhalten und zu verbessern. Die Erwartung der Anwenderinnen und Anwender ist groß. Oft wird eine zusätzliche Wirkung neben der hervorragenden Pflegewirkung erwartet. Diese zusätzliche Wirkung kann in einer verbesserten Rheologie oder einer sensorischen Wirkung liegen.

Es war die Aufgabe der vorliegenden Erfindung, eine kosmetische Zusammensetzung bereitzustellen, bei der die Nachhaltigkeit bei der Auswahl der Rohstoffe gesteigert ist, die jedoch ein hohes Maß an Leistungsfähigkeit hinsichtlich der Pflegewirkung zeigt. Die kosmetische Zusammensetzung soll Rohstoffe aus natürlichen Quellen oder Rohstoffe beinhalten, die eine natürliche Basis haben, oder Rohstoffe beinhalten, die mindestens teilweise biologisch abbaubar sind. Insbesondere lag der vorliegenden Erfindung die Aufgabe zugrunde, eine kosmetische Zusammensetzung bereitzustellen, die frei ist von Silizium enthaltenden organischen Verbindungen, und die dem Anwender bzw. der Anwenderin ein hohes Maß an Pflegeempfinden nach der Anwendung bietet.

Gelöst wird die Aufgabe durch die silikonfreie kosmetische Zusammensetzung gemäß Anspruch 1. Die Aufgabe wird gelöst durch eine silikonfreie kosmetische Zusammensetzung zur Pflege keratinischen Materials, insbesondere zur Pflege menschlicher Haare, die bezogen auf das Gesamtgewicht der silikonfreien kosmetischen Zusammensetzung a) 5 bis 40 Gew.-% eines natürlichen Wachses und b) 4 bis 25 Gew.-% einer Ölkomponente umfasst.

Überraschenderweise wurde gefunden, dass die hohe Konzentration an Wachs in Kombination mit der Ölkomponente eine hervorragende Pflegeleistung gewährleistet. Dank der hochkonzentrierten Wachse trocknet die Zusammensetzung nach 15 Minuten Anwendung auf dem Haar. Dies führt zu einem neuen Verwöhnerlebnis mit Cocoon-Effekt zusätzlich zu einer hohen Pflegeleistung. Die Nachhaltigkeit der kosmetischen Zusammensetzung wird insbesondere dadurch gewährleistet, dass diese silikonfrei ist. Darunter wird im Rahmen der Offenbarung verstanden, dass im Wesentlichen keine organischen Komponenten enthalten sind, die Siliciumatome enthalten. "Im Wesentlichen" bedeutet, dass keine solche Komponenten hinzugefügt werden. Bevorzugt bedeutet "im Wesentlichen", dass die Konzentration von Silicium enthaltenden Verbindungen unter 0,1 Gew.-% liegt.

Unter einem natürlichen Wachs wird im Rahmen der Offenbarung ein Wachs verstanden, das aus einer natürlichen Quelle stammt. Dabei handelt es sich bevorzugt um pflanzliche oder tierische Wachse. Gemäß einer bevorzugten Ausführungsform ist das natürliche Wachs ausgewählt aus der Gruppe bestehend aus Bienenwachs (INCI: Beeswax Cera Alba), Wollwachs (INCI: Lanolin), Chinawachs, Reiskleiwachs, Ouricorywachs, Karnaubawachs (INCI: Copernicia Cerifera Cera), Candelillawachs, Beerenwachs (INCI Rhus Verniciflua Peel Cera), Espartograswachs, Myrica Cerifera Fruit Wax (INCI), Schellackwachs, Japanwachs, Sumachwachs, Sonnenblumenwachs und Kombinationen davon. Das natürliche Wachs ist bevorzugt Bienenwachs, insbesondere das Bienenwachs Beeswax Cera Alba gemäß der Bezeichnung nach INCI. Das gilt für die übrigen Wachse entsprechend.

Das Wachs ist gemäß einer weiteren bevorzugten Ausführungsform ein natürliches Wachs, das ein Wachs umfasst, das ein Produkt ist aus einem dreiwertigen Alkohol, der mit einer oder verschiedenen gesättigten oder ungesättigten, verzweigten oder unverzweigten Fettsäuren mit 16 bis 32, bevorzugt 18 mit 28, bevorzugter 20 bis 26 Kohlenstoffatomen vollständig oder teilweise, bevorzugt vollständig, verestert ist.

Es ist vorteilhaft für die Lösung der obigen Aufgaben, wenn die organischen Reste aus den Fettsäureanteilen bei dem verwendeten Wachs Kettenlängen in den obigen Bereichen aufweisen. Für die oben genannten natürlichen Wachse ist dies überwiegend gegeben.

Vorteilhafterweise ist auch die erfindungsgemäße Ölkomponente natürlichen Ursprungs. Gemäß einer bevorzugten Ausführungsform besteht die Ölkomponente, bezogen auf das Gesamtgewicht der Ölkomponente, zu mehr als 30 Gew.-%, bevorzugt mehr als 50 Gew.-%, bevorzugter mehr als 70 Gew.-%, noch bevorzugter mehr als 90 Gew.-% aus einem natürlichen oder einem nativen Öl.

Unter einem natürlichen Öl wird im Rahmen der Offenbarung ein Öl verstanden, das aus einer natürlichen Quelle stammt. Dabei handelt es sich bevorzugt um pflanzliche oder tierische Öle. Die natürlichen Öle können auch modifizierte natürliche Öle, bevorzugt hydrierte Öle darstellen.

Unter einem nativen Öl wird ein kaltgepresstes Öl verstanden. Dessen Vorteil ist die hohe Qualität. Durch die schonende Gewinnung bleiben Vitamine und die als gesund geltenden, mehrfach ungesättigten Fettsäuren erhalten. Ferner ist die Kaltpressung zur Gewinnung der Öle vor einem energetischen Gesichtspunkt nachhaltiger. Auf diese Weise wird die obige Aufgabe der höheren Nachhaltigkeit erreicht.

Für das Lösen der Aufgabe einer hohen Leistungsfähigkeit im Sinne der kosmetischen Eigenschaften ist die Verwendung von ausschließlich natürlichen Ölen nicht einfach. Der Anteil der natürlichen Öle oder der nativen Öle soll so hoch wie möglich gewählt werden, ohne die kosmetischen Eigenschaften zu verschlechtern. Gemäß bevorzugter Ausführungsformen liegt aus diesem Grund der Anteil in den oben gewählten Bereichen.

Gemäß bevorzugter Ausführungsformen umfasst das natürliche Öl oder das native Öl Apricot Kernel (Aprikosen)-Kernöl, Arnica Montana (Arnica)-Öl, Calendulaöl, Vitis Vinifera (Trauben)-Kernöl, Cocos Nucifera (Kokosnuss)-Öl, Prunus Amygdalus Dulcis (Süßmandel)-Öl, Juglans Regia (Walnuss)-Kernöl, Prunus Persica (Pfirsich)-Kernöl, Persea Gratissima (Avocado)-Öl, Melaleuca Alternifolia (Teebaum)-Blattöl, Glycin Soja (Sojabohnen)-Öl, Gossypium Herbaceum (Baumwoll)-Samenöl, Sesamum Indicum (Sesam)-Samenöl, Helianthus Annuus (Sonnenblumen)-Samenöl, Camellia Japonica Samenöl (Tsubakiöl), Oenothera Biennis (Nachtkerzen)-Öl, Oryza Sativa (Reis)-Kleieöl, Elaeis Guineensis (Palm)-Öl, Mangifera Indica (Mango)-Kernöl, Vaccinium Macrocarpon (Cranberry)-Kernöl, Hippophae Rhamnoides Fruchtöl, Wiesenschaumkraut-Öl, Carthamus Tinctorius (Saflor)-Samenöl, Macadamia Ternifolia Kernöl, Amaranthus Spinosus Samenöl, Argania Spinosa Kernöl, Bambusöl, Olea Europaea (Oliven)-Fruchtöl, Triticum Vulgare (Weizen)-Keimöl, Cucurbita Pepo (Kürbis)-Kernöl, Neemöl, Malvenöl, Corylus Americana (Haselnuss)-Kernöl, Zea Mays (Mais)-Öl, Brassica Campestris (Raps)-Samenöl, Rapsöl, Sasanqua-Öl, Simmondsia Chinensis (Jojoba)-Kernöl, Rizinusöl, Rambutanöl, Sclerocarya Birrea Samenöl, Chenopodium Quinoa Samenöl oder Mischungen davon, insbesondere Apricot Kernel (Aprikosen)-Kernöl. Diese Öle tragen besonders zur Pflegewirkung, insbesondere zum subjektiven Pflegeempfinden bei.

Neben den natürlichen oder nativen Ölen können die silikonfreien kosmetischen Zusammensetzungen Öle umfassen, die nicht unter die Kategorien der nativen oder natürlichen Öle fallen. Gemäß bevorzugter Ausführungsformen enthält die kosmetische Zusammensetzung ein Öl, das zur Ölkomponente gehört, das ausgewählt ist aus der Gruppe bestehend aus linearen, gesättigten 1-Alkanolen mit 8 bis 22, bevorzugt 9 bis 18, bevorzugter 10 bis 16 Kohlenstoffatomen, Estern aus einer gesättigten oder ungesättigten Fettsäure umfassend 4 bis 14, bevorzugt 6 bis 12, bevorzugter 8 bis 10 Kohlenstoffatome mit einem verzweigten oder unverzweigten, gesättigten oder ungesättigtem Alkohol umfassend 3 bis 12, bevorzugt 4 bis 10, bevorzugter 5 bis 8 Kohlenstoffatome, Mineralöl, Paraffinöl und C₁₈-C₃₀-Isoparaffinen.

Die silikonfreien kosmetischen Zusammensetzungen können weitere Inhaltsstoffe umfassen. Insbesondere können bevorzugte silikonfreie kosmetische Zusammensetzungen, bezogen auf das Gesamtgewicht der silikonfreien kosmetischen Zusammensetzung, c) 0,1 bis 15 Gew.-% einer Pflegekomponente umfassen.

Vorteilhafte Pflegekomponenten können unterschiedlicher Natur sein. Gemäß bevorzugter Ausführungsformen ist die Pflegekomponenten eine natürliche Pflegekomponente, bevorzugt eine kationische Pflegekomponente auf natürlicher Basis, oder die Pflegekomponente ist ausgewählt aus der Gruppe bestehend aus
i) quarternären Imidazolinen der Formel, 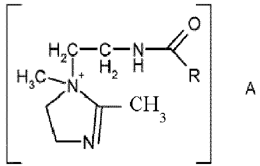 in welcher der Rest R für einen gesättigten oder ungesättigten, linearen oder verzweigten Kohlenwasserstoffrest mit einer Kettenlänge von 8 bis 30 Kohlenstoffatomen und A für ein physiologisch verträgliches Anion steht, bevorzugt Quaternium-27, Quaternium-83 oder Quaternium-87, insbesondere Quaternium-87,
ii) Aminen und/oder kationisierten Aminen,
iii) quaternisierten Cellulose-Derivaten, insbesondere Polyquaternium 10, Polyquaternium-24, Polyquaternium-27, Polyquaternium-67, Polyquaternium-72,
iv) kationischen Alkylpolyglycosiden,
v) kationisiertem Honig,
vi) kationischen Guar-Derivaten,
vii) Chitosan,
viii) Alkylamidoaminen, bevorzugt Stearamidopropyldimethylamin, Cocamidoproypyldimethylamin, Ricinolamidopropyldimethylamin, Isostearamidopropyldimethylamin, Oleamidopropyldimethylamin, Behenamidopropyldimethylamin, Palmamidopropyldimethylamin, Quaternium-33, Behenamidopropyl Ethyldimonium Ethosulfate, Brassicamidopropyldimethylamin oder Kombinationen davon, insbesondere Stearamidopropyldimethylamin, und
ix) Kombinationen davon. Die genannten Pflegekomponenten basieren entweder aus natürlichen Quellen, oder es ist mindestens in Teilen eine vorteilhafte biologische Abbaubarkeit gegeben.

Ferner können die silikonfreien kosmetischen Zusammensetzungen, bezogen auf das Gesamtgewicht der silikonfreien kosmetischen Zusammensetzung, d) 0,5 bis 8 Gew.-% eines amphoteren, zwitterionischen oder nicht-ionischen Tensids, bevorzugt eines nicht-ionischen Tensids enthalten. Gemäß einer bevorzugten Ausführungsform ist das nicht-ionische Tensid ausgewählt aus einem alkoxylierten Fettalkohol, bevorzugt einem alkoxylierten Fettalkohol, das ein Anlagerungsprodukt von 2 bis 50 Mol Ethylenoxid an lineare Fettalkohole mit 12 bis 18 Kohlenstoffatomen oder ein Anlagerungsprodukt von 2 bis 50 Mol Ethylenoxid und 1 bis 5 Mol Propylenoxid an lineare Fettalkohole mit 12 bis 18 Kohlenstoffatomen ist, Gemischen der Anlagerungsprodukte, PEG-40-hydriertem Rizinusöl oder PEG-7-Glycerylcocoat.

Es ist ferner bevorzugt, dass die silikonfreie kosmetische Zusammensetzung ein oder mehrere Lösungsmittel enthält. Gemäß bevorzugter Ausführungsformen enthält die silikonfreie kosmetische Zusammensetzung Wasser oder ein Polyol oder Wasser und ein Polyol. Gemäß einer bevorzugten Ausführungsform umfasst die silikonfreie kosmetische Zusammensetzung, bezogen auf das Gesamtgewicht der silikonfreien kosmetischen Zusammensetzung, e) 3 bis 18 Gew.-%, bevorzugt 5 bis 15, bevorzugter 8 bis 12 Gew.-% mindestens eines Polyols. Das Polyol kann ausgewählt sein aus 1,2-Propylenglycol, 1,3- Propylenglycol, 1,3-Butylenglycol, Ethylenglycol, Dipropylenglycol, Glycerin und/oder Diglycerin, wobei Glycerin besonders bevorzugt ist.

Die Menge an Wasser liegt bevorzugt, bezogen auf das Gesamtgewicht der silikonfreien kosmetischen Zusammensetzung, bei einer Menge von 10 bis 80 Gew.-%, bevorzugt von 20 bis 70 Gew.-%, bevorzugter von 30 bis 60 Gew.-%, am meisten bevorzugt von 40 bis 50 Gew.-%.

Die der Erfindung zugrunde liegende Aufgabe wird ferner gelöst durch die Verwendung der silikonfreien kosmetischen Zusammensetzung zur Pflege keratinischer Fasern, insbesondere Haare. Bevorzugt wird die silikonfreie kosmetische Zusammensetzung zur Erzeugung des Cocoon-Effekts verwendet.

Die der Erfindung zugrunde liegende Aufgabe wird ferner gelöst durch ein Verfahren, bei dem die silikonfreie kosmetische Zusammensetzung auf die Haare aufgetragen wird und dort einwirken gelassen wird für 5 Sekunden bis 30 Minuten, bevorzugt 15 Sekunden bis 15 Minuten, bevorzugt 60 Sekunden bis 5 Minuten. Alternativ wird die der Erfindung zugrunde liegende Aufgabe ferner gelöst durch ein Verfahren, bei dem die silikonfreie kosmetische Zusammensetzung auf die Haare aufgetragen und dort belassen wird.

Selbstverständlich können Merkmale, die nur im Zusammenhang mit der hier beschriebenen silikonfreien kosmetischen Zusammensetzung offenbart sind, als Merkmale für das hier beschriebene Verfahren oder für die hier beschriebenen Verwendungen dienen.

Die Erfindung kann auch durch die nachfolgenden Aussagen definiert werden:
1. Silikonfreie kosmetische Zusammensetzung zur Pflege keratinischen Materials, insbesondere zur Pflege menschlicher Haare, umfassend bezogen auf das Gesamtgewicht der silikonfreien kosmetischen Zusammensetzung
   a) 5 bis 40 Gew.-% eines natürlichen Wachses,
   b) 4 bis 25 Gew.-% einer Ölkomponente.
2. Silikonfreie kosmetische Zusammensetzung nach Aussage 1, umfassend bezogen auf das Gesamtgewicht der silikonfreien kosmetischen Zusammensetzung
   a) 6 bis 30 Gew.-% eines natürlichen Wachses,
   b) 5 bis 20 Gew.-% einer Ölkomponente.
3. Silikonfreie kosmetische Zusammensetzung nach Aussage 1 oder Aussage 2, umfassend bezogen auf das Gesamtgewicht der silikonfreien kosmetischen Zusammensetzung
   a) 7 bis 25 Gew.-% eines natürlichen Wachses,
   b) 6 bis 18 Gew.-% einer Ölkomponente.
4. Silikonfreie kosmetische Zusammensetzung nach einer der vorangehenden Aussagen, umfassend bezogen auf das Gesamtgewicht der silikonfreien kosmetischen Zusammensetzung
   a) 8 bis 20 Gew.-% eines natürlichen Wachses,
   b) 7 bis 15 Gew.-% einer Ölkomponente.
5. Silikonfreie kosmetische Zusammensetzung nach einer der vorangehenden Aussagen, ferner umfassend bezogen auf das Gesamtgewicht der silikonfreien kosmetischen Zusammensetzung
   c) 0,1 bis 15 Gew.-% einer Pflegekomponente.
6. Silikonfreie kosmetische Zusammensetzung nach einer der vorangehenden Aussagen, ferner umfassend bezogen auf das Gesamtgewicht der silikonfreien kosmetischen Zusammensetzung
   c) 1 bis 10 Gew.-% einer Pflegekomponente.
7. Silikonfreie kosmetische Zusammensetzung nach einer der vorangehenden Aussagen, ferner umfassend bezogen auf das Gesamtgewicht der silikonfreien kosmetischen Zusammensetzung
   c) 2 bis 8 Gew.-% einer Pflegekomponente.
8. Silikonfreie kosmetische Zusammensetzung nach einer der vorangehenden Aussagen, ferner umfassend bezogen auf das Gesamtgewicht der silikonfreien kosmetischen Zusammensetzung
   c) 4 bis 6 Gew.-% einer Pflegekomponente.
9. Silikonfreie kosmetische Zusammensetzung nach einer der Aussagen 5 bis 8, dadurch gekennzeichnet, dass die Pflegekomponente eine natürliche Pflegekomponente, bevorzugt eine kationische Pflegekomponente auf natürlicher Basis ist.
10. Silikonfreie kosmetische Zusammensetzung nach einer der Aussagen 5 bis 9, dadurch gekennzeichnet, dass die Pflegekomponente ausgewählt ist aus der Gruppe bestehend aus
   i) quarternären Imidazolinen der Formel, 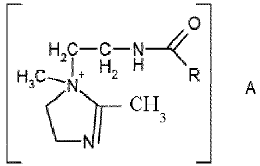 in welcher der Rest R für einen gesättigten oder ungesättigten, linearen oder verzweigten Kohlenwasserstoffrest mit einer Kettenlänge von 8 bis 30 Kohlenstoffatomen und A für ein physiologisch verträgliches Anion steht, bevorzugt Quaternium-27, Quaternium-83 oder Quaternium-87, insbesondere Quaternium-87,
   ii) Aminen und/oder kationisierten Aminen,
   iii) quaternisierten Cellulose-Derivaten, insbesondere Polyquaternium 10, Polyquaternium-24, Polyquaternium-27, Polyquaternium-67, Polyquaternium-72,
   iv) kationischen Alkylpolyglycosiden,
   v) kationisiertem Honig,
   vi) kationischen Guar-Derivaten,
   vii) Chitosan,
   viii) Alkylamidoaminen, bevorzugt Stearamidopropyldimethylamin, Cocamidoproypyldimethylamin, Ricinolamidopropyldimethylamin, Isostearamidopropyldimethylamin, Oleamidopropyldimethylamin, Behenamidopropyldimethylamin, Palmamidopropyldimethylamin, Quaternium-33, Behenamidopropyl Ethyldimonium Ethosulfate, Brassicamidopropyldimethylamin oder Kombinationen davon, insbesondere Stearamidopropyldimethylamin, und
   ix) Kombinationen davon.
11. Silikonfreie kosmetische Zusammensetzung nach einer der Aussagen 5 bis 10, dadurch gekennzeichnet, dass die Pflegekomponente ausgewählt ist aus der Gruppe bestehend aus Quaternium-27, Quaternium-83, Quaternium-87, Stearamidopropyldimethylamin, Cocamidoproypyldimethylamin und Kombinationen davon.
12. Silikonfreie kosmetische Zusammensetzung nach einer der vorangehenden Aussagen, dadurch gekennzeichnet, dass das natürliche Wachs ausgewählt ist aus der Gruppe bestehend aus Bienenwachs (INCI: Beeswax Cera Alba), Wollwachs (INCI: Lanolin), Chinawachs, Reiskleiwachs, Ouricorywachs, Karnaubawachs (INCI: Copernicia Cerifera Cera), Candelillawachs, Beerenwachs (INCI Rhus Verniciflua Peel Cera), Espartograswachs, Myrica Cerifera Fruit Wax (INCI), Schellackwachs, Japanwachs, Sumachwachs, Sonnenblumenwachs und Kombinationen davon.
13. Silikonfreie kosmetische Zusammensetzung nach einer der vorangehenden Aussagen, dadurch gekennzeichnet, dass das natürliche Wachs ausgewählt ist aus der Gruppe bestehend aus Bienenwachs, Wollwachs, Karnaubawachs, Beerenwachs und Kombinationen davon.
14. Silikonfreie kosmetische Zusammensetzung nach einer der vorangehenden Aussagen, dadurch gekennzeichnet, dass das natürliche Wachs ein Wachs umfasst, das ein Produkt ist aus einem dreiwertigen Alkohol, der mit einer oder verschiedenen gesättigten oder ungesättigten, verzweigten oder unverzweigten Fettsäuren mit 16 bis 32, bevorzugt 18 mit 28, bevorzugter 20 bis 26 Kohlenstoffatomen vollständig oder teilweise verestert ist.
15. Silikonfreie kosmetische Zusammensetzung nach einer der vorangehenden Aussagen, dadurch gekennzeichnet, dass die Ölkomponente, bezogen auf das Gesamtgewicht der Ölkomponente, zu mehr als 30 Gew.-%, bevorzugt mehr als 50 Gew.-%, bevorzugter mehr als 70 Gew.-%, noch bevorzugter mehr als 90 Gew.-% aus einem natürlichen Öl besteht.
16. Silikonfreie kosmetische Zusammensetzung nach einer der vorangehenden Aussagen, dadurch gekennzeichnet, dass die Ölkomponente, bezogen auf das Gesamtgewicht der Ölkomponente, zu mehr als 30 Gew.-%, bevorzugt mehr als 50 Gew.-%, bevorzugter mehr als 70 Gew.-%, noch bevorzugter mehr als 90 Gew.-% aus einem nativen Öl besteht.
17. Silikonfreie kosmetische Zusammensetzung nach einer der vorangehenden Aussagen, dadurch gekennzeichnet, dass das natürliche Öl oder das native Öl Apricot Kernel (Aprikosen)-Kernöl, Arnica Montana (Arnica)-Öl, Calendulaöl Vitis Vinifera (Trauben)-Kernöl, Cocos Nucifera (Kokosnuss)-Öl, Prunus Amygdalus Dulcis (Süßmandel)-Öl, Juglans Regia (Walnuss)-Kernöl, Prunus Persica (Pfirsich)-Kernöl, Persea Gratissima (Avocado)-Öl, Melaleuca Alternifolia (Teebaum)-Blattöl, Glycin Soja (Sojabohnen)-Öl, Gossypium Herbaceum (Baumwoll)-Samenöl, Sesamum Indicum (Sesam)-Samenöl, Helianthus Annuus (Sonnenblumen)-Samenöl, Camellia Japonica Samenöl (Tsubakiöl), Oenothera Biennis (Nachtkerzen)-Öl, Oryza Sativa (Reis)-Kleieöl, Elaeis Guineensis (Palm)-Öl, Mangifera Indica (Mango)-Kernöl, Vaccinium Macrocarpon (Cranberry)-Kernöl, Hippophae Rhamnoides Fruchtöl, Wiesenschaumkraut-Öl, Carthamus Tinctorius (Saflor)-Samenöl, Macadamia Ternifolia Kernöl, Amaranthus Spinosus Samenöl, Argania Spinosa Kernöl (Arganöl), Bambusöl, Olea Europaea (Oliven)-Fruchtöl, Triticum Vulgare (Weizen)-Keimöl, Cucurbita Pepo (Kürbis)-Kernöl, Neemöl, Malvenöl, Corylus Americana (Haselnuss)-Kernöl, Zea Mays (Mais)-Öl, Brassica Campestris (Raps)-Samenöl, Rapsöl, Sasanqua-Öl, Simmondsia Chinensis (Jojoba)-Kernöl, Rizinusöl, Rambutanöl, Sclerocarya Birrea Samenöl, Chenopodium Quinoa Samenöl oder Mischungen davon, insbesondere Apricot Kernel (Aprikosen)-Kernöl, umfasst.
18. Silikonfreie kosmetische Zusammensetzung nach einer der vorangehenden Aussagen, dadurch gekennzeichnet, dass das natürliche Öl oder das native Öl Aprikosen-Kernöl, Arnica Montana (Arnica)-Öl, Sonnenblumen-Samenöl, Nachtkerzen-Öl, Arganöl, Oliven-Fruchtöl, Neemöl, Rapsöl, Jojoba-Kernöl, Rizinusöl oder Mischungen davon umfasst.
19. Silikonfreie kosmetische Zusammensetzung nach einer der vorangehenden Aussagen, dadurch gekennzeichnet, dass die Ölkomponente ein Öl umfasst, das ausgewählt ist aus der Gruppe bestehend aus linearen, gesättigten 1-Alkanolen mit 8 bis 22, bevorzugt 9 bis 18, bevorzugter 10 bis 16 Kohlenstoffatomen, Estern aus einer gesättigten oder ungesättigten Fettsäure umfassend 4 bis 14, bevorzugt 6 bis 12, bevorzugter 8 bis 10 Kohlenstoffatome mit einem verzweigten oder unverzweigten, gesättigten oder ungesättigtem Alkohol umfassend 3 bis 12, bevorzugt 4 bis 10, bevorzugter 5 bis 8 Kohlenstoffatome, Mineralöl, Paraffinöl und C₁₈-C₃₀-Isoparaffinen.
20. Silikonfreie kosmetische Zusammensetzung nach einer der vorangehenden Aussagen, ferner umfassend bezogen auf das Gesamtgewicht der silikonfreien kosmetischen Zusammensetzung
   d) 0,1 bis 8 Gew.-% eines amphoteren, zwitterionischen oder nicht-ionischen Tensids, bevorzugt eines nicht-ionischen Tensids.
21. Silikonfreie kosmetische Zusammensetzung nach einer der Aussagen 1 bis 19, ferner umfassend bezogen auf das Gesamtgewicht der silikonfreien kosmetischen Zusammensetzung
   d) 0,5 bis 6 Gew.-% eines amphoteren, zwitterionischen oder nicht-ionischen Tensids, bevorzugt eines nicht-ionischen Tensids.
22. Silikonfreie kosmetische Zusammensetzung nach einer der Aussagen 1 bis 19, ferner umfassend bezogen auf das Gesamtgewicht der silikonfreien kosmetischen Zusammensetzung
   d) 1 bis 4 Gew.-% eines amphoteren, zwitterionischen oder nicht-ionischen Tensids, bevorzugt eines nicht-ionischen Tensids.
23. Silikonfreie kosmetische Zusammensetzung nach einer der Aussagen 1 bis 19, ferner umfassend bezogen auf das Gesamtgewicht der silikonfreien kosmetischen Zusammensetzung
   d) 1,3 bis 3,8 Gew.-% eines amphoteren, zwitterionischen oder nicht-ionischen Tensids, bevorzugt eines nicht-ionischen Tensids.
24. Silikonfreie kosmetische Zusammensetzung nach einer der Aussagen 20 bis 23, dadurch gekennzeichnet, dass das nicht-ionische Tensid ausgewählt ist aus einem alkoxylierten Fettalkohol, bevorzugt einem alkoxylierten Fettalkohol, das ein Anlagerungsprodukt von 2 bis 50 Mol Ethylenoxid an lineare Fettalkohole mit 12 bis 18 Kohlenstoffatomen oder ein Anlagerungsprodukt von 2 bis 50 Mol Ethylenoxid und 1 bis 5 Mol Propylenoxid an lineare Fettalkohole mit 12 bis 18 Kohlenstoffatomen ist, Gemischen der Anlagerungsprodukte, PEG-40-hydriertem Rizinusöl oder PEG-7-Glycerylcocoat.
25. Silikonfreie kosmetische Zusammensetzung nach einer der Aussagen 20 bis 23, dadurch gekennzeichnet, dass das zwitterionische Tensid ausgewählt ist aus Cocamidopropyl Betaine oder Cocobetaine.
26. Silikonfreie kosmetische Zusammensetzung nach einer der Aussagen 20 bis 23, dadurch gekennzeichnet, dass die silikonfreie kosmetische Zuammensetzung ein zwitterionisches und ein nicht-ionisches Tensid enthält.
27. Silikonfreie kosmetische Zusammensetzung nach einer der vorangehenden Aussagen, ferner umfassend bezogen auf das Gesamtgewicht der silikonfreien kosmetischen Zusammensetzung
   e) 3 bis 18 Gew.-%, bevorzugt 5 bis 15, bevorzugter 8 bis 12 Gew.-% mindestens eines Polyols.
28. Silikonfreie kosmetische Zusammensetzung nach Aussage 27, dadurch gekennzeichnet, dass das Polyol ausgewählt aus 1,2-Propylenglycol, 1,3- Propylenglycol, 1,3-Butylenglycol, Ethylenglycol, Dipropylenglycol, Glycerin und/oder Diglycerin, besonders bevorzugt Glycerin.
29. Silikonfreie kosmetische Zusammensetzung nach einer der vorangehenden Aussagen, ferner umfassend Wasser, bezogen auf das Gesamtgewicht der silikonfreien kosmetischen Zusammensetzung, in einer Menge von 10 bis 80 Gew.-%, bevorzugt von 20 bis 70 Gew.-%, bevorzugter von 30 bis 60 Gew.-%, am meisten bevorzugt von 40 bis 50 Gew.-%.
30. Verwendung einer kosmetischen Zusammensetzung gemäß einer der Aussagen 1 bis 29 zur Pflege von keratinischem Material, insbesondere zur Pflege menschlicher Haare.
31. Verfahren zur Pflege keratinischer Fasern, insbesondere menschlicher Haare, bei dem die silikonfreie kosmetische Zusammensetzung gemäß einer der Aussagen 1 bis 29 auf die Haare aufgetragen wird und wieder ausgespült wird.
32. Verfahren zur Pflege keratinischer Fasern, insbesondere menschlicher Haare, bei dem die silikonfreie kosmetische Zusammensetzung gemäß einer der Aussagen 1 bis 29 auf die Haare aufgetragen wird und dort belassen wird.

### Beispiele

1. Haarpflegezusammensetzung

| | Gew.-% |
|---|---|
| Triglycerid von Behensäure | 6 |
| Beerenwachs | 7,5 |
| Natural Vaseline | 1,5 |
| Apricot kernel oil | 3,8 |
| Cetearylalkohol | 4 |
| Ceteareth-20 | 5 |
| Stearamidopropyl Dimethylamine | 0,5 |
| Glycerin | 12 |
| Isopropylmyristat | 2 |
| Imidazoline quat | 4 |
| Konservierungsmittel, Parfum | q.s. |
| Wasser, demineralisiert | Rest |

2. Haarpflegezusammensetzung

| | Gew.-% |
|---|---|
| Triglycerid von Behensäure | 8 |
| Carnaubauwachs | 8,5 |
| Natural Vaseline | 1,5 |
| Argania Spinosa Kernöl | 3,8 |
| Cetearylalkohol | 6 |
| Ceteareth-20 | 5 |
| Stearamidopropyl Dimethylamine | 0,5 |
| Glycerin | 12 |
| Isopropylmyristat | 2 |
| Imidazoline quat | 4 |
| Milchsäure | 1 |
| Konservierungsmittel, Parfum | q.s. |
| Wasser, demineralisiert | Rest |

Die Komponenten gemäß der obigen Tabelle werden zusammengegeben und gemischt. Das entstandene Gemisch wird auf die Haare aufgetragen und etwa 2 Min. einmassiert. Anschließend wird das Gemisch mit Wasser ausgespült.

## Patentansprüche

1. Silikonfreie kosmetische Zusammensetzung zur Pflege keratinischen Materials, insbesondere zur Pflege menschlicher Haare, umfassend bezogen auf das Gesamtgewicht der silikonfreien kosmetischen Zusammensetzung
a) 5 bis 40 Gew.-% eines natürlichen Wachses,
b) 4 bis 25 Gew.-% einer Ölkomponente.

2. Silikonfreie kosmetische Zusammensetzung nach Anspruch 1, ferner umfassend bezogen auf das Gesamtgewicht der silikonfreien kosmetischen Zusammensetzung c) 0,1 bis 15 Gew.-% einer Pflegekomponente.

3. Silikonfreie kosmetische Zusammensetzung nach einem der Ansprüche 1 bis 3, **dadurch gekennzeichnet, dass** die Pflegekomponente eine natürliche Pflegekomponente, bevorzugt eine kationische Pflegekomponente auf natürlicher Basis ist, oder wobei die Pflegekomponente ausgewählt ist aus der Gruppe bestehend aus
i) quarternären Imidazolinen der Formel, 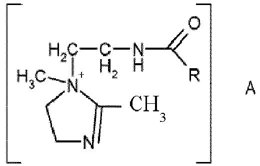 in welcher der Rest R für einen gesättigten oder ungesättigten, linearen oder verzweigten Kohlenwasserstoffrest mit einer Kettenlänge von 8 bis 30 Kohlenstoffatomen und A für ein physiologisch verträgliches Anion steht, bevorzugt Quaternium-27, Quaternium-83 oder Quaternium-87, insbesondere Quaternium-87,
ii) Aminen und/oder kationisierten Aminen,
iii) quaternisierten Cellulose-Derivaten, insbesondere Polyquaternium 10, Polyquaternium-24, Polyquaternium-27, Polyquaternium-67, Polyquaternium-72,
iv) kationischen Alkylpolyglycosiden,
v) kationisiertem Honig,
vi) kationischen Guar-Derivaten,
vii) Chitosan,
viii) Alkylamidoaminen, bevorzugt Stearamidopropyldimethylamin, Cocamidoproypyldimethylamin, Ricinolamidopropyldimethylamin, Isostearamidopropyldimethylamin, Oleamidopropyldimethylamin, Behenamidopropyldimethylamin, Palmamidopropyldimethylamin, Quaternium-33, Behenamidopropyl Ethyldimonium Ethosulfate, Brassicamidopropyldimethylamin oder Kombinationen davon, insbesondere Stearamidopropyldimethylamin, und
ix) Kombinationen davon.

4. Silikonfreie kosmetische Zusammensetzung nach einem der vorangehenden Ansprüche, **dadurch gekennzeichnet, dass** das natürliche Wachs ausgewählt ist aus der Gruppe bestehend aus Bienenwachs (INCI: Beeswax Cera Alba), Wollwachs (INCI: Lanolin), Chinawachs, Reiskleiwachs, Ouricorywachs, Karnaubawachs (INCI: Copernicia Cerifera Cera), Candelillawachs, Beerenwachs (INCI Rhus Verniciflua Peel Cera), Espartograswachs, Myrica Cerifera Fruit Wax (INCI), Schellackwachs, Japanwachs, Sumachwachs, Sonnenblumenwachs und Kombinationen davon.

5. Silikonfreie kosmetische Zusammensetzung nach einem der vorangehenden Ansprüche, **dadurch gekennzeichnet, dass** das natürliche Wachs ein Wachs umfasst, das ein Produkt ist aus einem dreiwertigen Alkohol, der mit einer oder verschiedenen gesättigten oder ungesättigten, verzweigten oder unverzweigten Fettsäuren mit 16 bis 32, bevorzugt 18 mit 28, bevorzugter 20 bis 26 Kohlenstoffatomen vollständig oder teilweise verestert ist.

6. Silikonfreie kosmetische Zusammensetzung nach einem der vorangehenden Ansprüche, **dadurch gekennzeichnet, dass** die Ölkomponente, bezogen auf das Gesamtgewicht der Ölkomponente, zu mehr als 30 Gew.-%, bevorzugt mehr als 50 Gew.-%, bevorzugter mehr als 70 Gew.-%, noch bevorzugter mehr als 90 Gew.-% aus einem natürlichen oder einem nativen Öl besteht.

7. Silikonfreie kosmetische Zusammensetzung nach einem der vorangehenden Ansprüche, **dadurch gekennzeichnet, dass** das natürliche Öl oder das native Öl Apricot Kernel (Aprikosen)-Kernöl, Arnica Montana (Arnica)-Öl, Calendulaöl, Vitis Vinifera (Trauben)-Kernöl, Cocos Nucifera (Kokosnuss)-Öl, Prunus Amygdalus Dulcis (Süßmandel)-Öl, Juglans Regia (Walnuss)-Kernöl, Prunus Persica (Pfirsich)-Kernöl, Persea Gratissima (Avocado)-Öl, Melaleuca Alternifolia (Teebaum)-Blattöl, Glycin Soja (Sojabohnen)-Öl, Gossypium Herbaceum (Baumwoll)-Samenöl, Sesamum Indicum (Sesam)-Samenöl, Helianthus Annuus (Sonnenblumen)-Samenöl, Camellia Japonica Samenöl (Tsubakiöl), Oenothera Biennis (Nachtkerzen)-Öl, Oryza Sativa (Reis)-Kleieöl, Elaeis Guineensis (Palm)-Öl, Mangifera Indica (Mango)-Kernöl, Vaccinium Macrocarpon (Cranberry)-Kernöl, Hippophae Rhamnoides Fruchtöl, Wiesenschaumkraut-Öl, Carthamus Tinctorius (Saflor)-Samenöl, Macadamia Ternifolia Kernöl, Amaranthus Spinosus Samenöl, Argania Spinosa Kernöl, Bambusöl, Olea Europaea (Oliven)-Fruchtöl, Triticum Vulgare (Weizen)-Keimöl, Cucurbita Pepo (Kürbis)-Kernöl, Neemöl, Malvenöl, Corylus Americana (Haselnuss)-Kernöl, Zea Mays (Mais)-Öl, Brassica Campestris (Raps)-Samenöl, Rapsöl, Sasanqua-Öl, Simmondsia Chinensis (Jojoba)-Kernöl, Rizinusöl, Rambutanöl, Sclerocarya Birrea Samenöl, Chenopodium Quinoa Samenöl oder Mischungen davon, insbesondere Apricot Kernel (Aprikosen)-Kernöl, umfasst.

8. Silikonfreie kosmetische Zusammensetzung nach einem der vorangehenden Ansprüche, **dadurch gekennzeichnet, dass** die Ölkomponente ein Öl umfasst, das ausgewählt ist aus der Gruppe bestehend aus linearen, gesättigten 1-Alkanolen mit 8 bis 22, bevorzugt 9 bis 18, bevorzugter 10 bis 16 Kohlenstoffatomen, Estern aus einer gesättigten oder ungesättigten Fettsäure umfassend 4 bis 14, bevorzugt 6 bis 12, bevorzugter 8 bis 10 Kohlenstoffatome mit einem verzweigten oder unverzweigten, gesättigten oder ungesättigtem Alkohol umfassend 3 bis 12, bevorzugt 4 bis 10, bevorzugter 5 bis 8 Kohlenstoffatome, Mineralöl, Paraffinöl und C₁₈-C₃₀-Isoparaffinen.

9. Silikonfreie kosmetische Zusammensetzung nach einem der vorangehenden Ansprüche, ferner umfassend bezogen auf das Gesamtgewicht der silikonfreien kosmetischen Zusammensetzung
d) 0,5 bis 8 Gew.-% eines amphoteren, zwitterionischen oder nicht-ionischen Tensids, bevorzugt eines nicht-ionischen Tensids.

10. Silikonfreie kosmetische Zusammensetzung nach Anspruch 9, **dadurch gekennzeichnet, dass** das nicht-ionische Tensid ausgewählt ist aus einem alkoxylierten Fettalkohol, bevorzugt einem alkoxylierten Fettalkohol, das ein Anlagerungsprodukt von 2 bis 50 Mol Ethylenoxid an lineare Fettalkohole mit 12 bis 18 Kohlenstoffatomen oder ein Anlagerungsprodukt von 2 bis 50 Mol Ethylenoxid und 1 bis 5 Mol Propylenoxid an lineare Fettalkohole mit 12 bis 18 Kohlenstoffatomen ist, Gemischen der Anlagerungsprodukte, PEG-40-hydriertem Rizinusöl oder PEG-7-Glycerylcocoat.
